(19) Europäisches Patentamt
European Patent Office
Office européen des brevets

(11) Veröffentlichungsnummer : **0 135 832**
**B1**

(12) # EUROPÄISCHE PATENTSCHRIFT

(45) Veröffentlichungstag der Patentschrift : **04.05.88**

(51) Int. Cl.⁴ : **C 08 G 18/38**, C 08 G 18/14, C 08 G 18/65, C 07 C127/15

(21) Anmeldenummer : **84110239.5**

(22) Anmeldetag : **29.08.84**

(54) **Verwendung von Poly-hydroxyalkyl-mono-harnstoffen für PU-Schaumstoffe, Dispersionen der Poly-hydroxyalkyl-mono-harnstoffe in Polyolen, sowie neue Tris-hydroxyalkyl-mono-harnstoffe.**

(30) Priorität : **10.09.83 DE 3332794**

(43) Veröffentlichungstag der Anmeldung : **03.04.85 Patentblatt 85/14**

(45) Bekanntmachung des Hinweises auf die Patenterteilung : **04.05.88 Patentblatt 88/18**

(84) Benannte Vertragsstaaten : **BE DE FR GB IT**

(56) Entgegenhaltungen :
**BE-A- 678 064**
**DE-A- 2 703 185**
**FR-A- 1 369 677**
**FR-A- 2 360 619**
**FR-A- 2 381 077**
**GB-A- 1 223 320**
**US-A- 4 180 631**

(73) Patentinhaber : **BAYER AG**
**Konzernverwaltung RP Patentabteilung**
**D-5090 Leverkusen 1 Bayerwerk (DE)**

(72) Erfinder : **Haas, Peter, Dr.**
**Zwengenberger Strasse 43**
**D-5657 Haan (DE)**
Erfinder : **Avar, Geza, Dr.**
**Bamberger Strasse 1**
**D-5090 Leverkusen (DE)**
Erfinder : **Grammes, Hartwig, Ing. grad**
**Reuterstrasse 14**
**D-5090 Leverkusen (DE)**

**EP 0 135 832 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind

1. Verwendung von Hydroxyalkylharnstoffen als einbaufähige Flammschutzmittel bei der Herstellung von flammgeschützten Polyurethanschaumstoffen, bei deren Herstellung Verbindungen mit gegenüber Isocyanatgruppen reaktionsfähigen Wasserstoffatomen des Molekulargewichtsbereichs 400-10 000 verwendet werden, wobei die Hydroxyalkylharnstoffe in einer Menge von 3 bis 100 Gewichtsteilen, bezogen auf 100 Gewichtsteile diese Verbindungen mit gegenüber Isocyanatgruppen reaktionsfähigen Gruppen eingesetzt werden, dadurch gekennzeichnet, daß man als Hydroxyalkylharnstoffe Tris-hydroxyalkyl-monoharnstoffe der allgemeinen Formel

$$HO-A-NH-\overset{\overset{O}{\|}}{C}-N(-A-OH)_2$$

verwendet, in welcher A für geradkettige oder verzweigte, gegebenenfalls eine oder mehrere OH-Gruppen aufweisende $C_2$-$C_6$-Alkylengruppen darstellt.

2. Dispersionen oder Suspensionen von Tris-hydroxyalkyl-mono-harnstoffen der allgemeinen Formel

$$HO-A-NH-\overset{\overset{O}{\|}}{C}-N(-A-OH)_2$$

in welcher A die obengenannte Bedeutung hat in höhermolekularen Verbindungen mit mindestens zwei gegenüber Isocyanatgruppen reaktionsfähigen Endgruppen aus der Reihe der Hydroxyl-, primären oder sekundären Amino-, Carboxyl- oder Hydrazid-Endgruppen mit Molekulargewichten von 400 bis 10000, wobei der Gehalt der Hydroxylalkylharnstoffe 3 bis 50 Gewichtsprozent ausmacht.

3. Tris-hydroxyalkyl-mono-harnstoffe der obengenannten allgemeinen Formel, für welche A die obengenannte Bedeutung hat mit Ausnahme von N,N-Bis-(2-hydroxy-propyl)-N'-(2-hydroxyethyl-mono-harnstoff).

Für den Einsatz von Polyurethanschaumstoffen ist eine weitgehende Resistenz gegenüber einer Entflammung für bestimmte Anwendungen von ausschlaggebender Bedeutung. Dies äußert sich in Normen zur Entflammbarkeit, die, abhängig vom jeweiligen Verwendungszweck, Unterschiede aufweisen können und oft auf spezielle Gesichtspunkte ausgerichtet sind. In diesem Zusammenhang sind der MVSS-302-Test, Bundesbahn-Test, Lufthansatest (FAR 25.853) sowie der Kleinbrenner-Test nach DIN 53438 zu sehen.

In diesem Zusammenhang auftretende Fragen zur Verwendung von Polyurethanschaumstoffen mit entsprechenden Anforderungen sind mit größter Aufmerksamkeit bearbeitet worden, was aus der Vielzahl des bisher vorliegenden Schrifttums zu diesem Thema zu ersehen ist.

Technisch von Interesse sind vor allem in Polyurethanschaumstoffen die Ester von Phosphor enthaltenden Säuren und deren Derivate.

Mit an erster Stelle sind hier Trischlorethylphosphat, dessen höhere Homologe und Trikresylphosphat, Derivate von Aminomethylphosphonsäureestern, Tris-(2,3-dibrompropyl)-phosphat sowie Brom enthaltende Buten- und Butandiole und deren höhermolekulare Oxiran-Addukte zu nennen ; aber auch Melamin- oder seine Phosphonsäurederivate sind bekannte Flammschutzzusätze.

Die Verwendung der aufgeführten Flammschutzmittel ist jedoch aus mehreren Gründen mit Nachteilen behaftet. Neben physiologischer Bedenken, vor allem bei β-Halogenestern des Phosphors, ist die oft unzureichende Stabilität gegen Hydrolyse dieser Substanzklassen als nachteilig bekannt geworden. Dies ist für die Verarbeiter der Polyurethanausgangskomponenten besonders unangenehm, da ja bekanntlich die Additionsreaktion durch tertiäre Aminogruppen enthaltende Verbindungen katalysiert wird. Wegen dieser, im basischen Bereich noch gesteigerten Instabilität der verwendeten Flammschutzmittel sind Formulierungen der Rohstoffe nur begrenzt lagerfähig. Mitunter werden sogar aus diesem Grunde in unmittelbar vor der Verarbeitung frisch hergestellten Formulierungen solche Aktivitätsveränderungen im Bereich mehrerer Stunden festgestellt. In der Regel ist dies dann nur noch schwer zu korrigieren ; die hergestellten Teile entfallen als Ausschuß, da eine Nachkatalyse problemreich ist.

Neben den bislang geschilderten, gewichtigen Nachteilen der verwendeten Flammschutzmittel ist noch deren Weichmacherwirkung zu nennen. Sie macht sich durch eine erhebliche Erniedrigung in der Wärmestandfestigkeit bemerkbar. Hinzu kommt durch die Flüchtigkeit der verwendeten Verbindungen ein Wirkungsverlust im Verlaufe der Zeit hinzu. Dieses Diffusionsvermögen äußert sich in Automobilen z. B. als wiederholt auftretende Abscheidung auf Glasflächen, vor allem der Windschutzscheiben (Fogging).

Aufgabe der vorliegenden Verbindung war es, einbaufähige Verbindungen mit flammschützender Wirkung für Polyurethanschaumstoffe zur Verfügung zu stellen, welche die geschilderten Nachteile des Standes der Technik, nämlich

— mangelnde Dispergierbarkeit oder Mischbarkeit und
— mangelnde Lagerbeständigkeit der mit Flammschutzmitteln ausgerüsteten Rohstoffmischung
— weichmachende Effekte des Flammschutzmittels
— schlechte Wärmestandfestigkeit des Schaumstoffs durch die Zusatzmittel
— Diffusionseffekte und Ausschwitzen des Flammschutzmittels, mithin also Wirkungsminderung und
— Abgabe von Halogenwasserstoffen bei Bränden,
nicht zeigen.

Überraschenderweise wurde jetzt gefunden, daß die vorstehend genannten Hydroxyalkyl-mono-harnstoffe hervorragend wirksame, einbaufähige Flammschutzmittel in PU-Schaumstoffen darstellen. Sie sind diesbezüglich den Hydroxyalkylharnstoffen, deren Verwendung in US-A 4 180 631 konkret beschrieben ist, eindeutig überlegen. Auch die allgemeinen Hinweise in FR-A-1 369 677, FR-A-2 381 077 und FR-A-2 360 619 auf die Verwendung von substituierten Harnstoffen für die Herstellung von Polyurethan-Schaumstoffen vermitteln keinen konkreten Hinweis auf die bevorzugte Eignung der erfindungsgemäß einzusetzenden Trishydroxyalkyl-mono-harnstoffe. Die erfindungsgemäß zu verwendenden Tris-hydroxyalkyl-mono-harnstoffe können in Form ihrer Dispersionen oder Suspensionen in höhermolekularen Verbindungen mit gegenüber NCO reaktiven Endgruppen, bevorzugt höhermolekularen Polyolen, zur Herstellung von PU-Schaumstoffen in an sich üblichen Verfahren der Schaumstoffherstellung, verwendet werden.

Die Herstellung der Tris-hydroxyalkyl-mono-harnstoffe erfolgt überlicherweise durch Umsetzung von Harnstoff und den entsprechenden Aminen, wie sie z. B. in US-PS 3 560 564, DE-PS 1 468 398, GB-PS 1 127 605 oder DE-OS 2 703 185 bereits beschrieben werden. Die Trishydroxyalkyl-mono-harnstoffe sind zumeist flüssige Substanzen, die sich in den Polyolen bei Raumtemperaturen stabil dispergieren lassen. Sie sind, mit Ausnahme des in DE-A-2 703 185 erwähnten N,N-Bis-(2-hydroxypropyl)-N'-(2-hydroxyethyl)-mono-harnstoff, in der Literatur bisher nicht beschrieben worden und werden daher als neue Verbindungen beansprucht.

Ein weiterer Erfindungsgegenstand sind auch die Dispersionen oder Suspensionen von Tris-hydroxyalkyl-mono-harnstoffen in höhermolekularen Verbindungen mit Hydroxyl-, primären oder sekundären Amino-, Carboxyl- oder Hydrazid-Endgruppen, vorzugsweise höhermolekularen Polyolen, besonders bevorzugt mehr als 2-funktionellen Polyethern, mit Molekulargewichten von 400-10.000, wobei ihr Gehalt 3-50, bevorzugt 10-30 Gew.-% der Monoharnstoffe in den höhermolekularen Verbindungen ausmacht. Besonders bevorzugt sind Dispersionen von Tris-hydroxyalkyl-mono-harnstoffen in höhermolekularen, mehr als 2-funktionellen Polyethern.

Auch in DE-PS 933783 bzw. 959679 sind bereits einige Harnstoffderivate als Reaktionskomponenten bei der Herstellung von Polyurethankunstoffen beschrieben. Sie werden jedoch lediglich als Kettenverlängerungsmittel bei der Herstellung von Polyurethan-Polyesterelastomeren werwendet, wobei Bis-hydroxyalkyl-mono-harnstoffe zum Einsatz gelangen, deren Verarbeitungstemperatur bei 100-130 °C liegt. Diese Harnstoffe haben jedoch den gewichtigen Nachteil, daß sie nach mehr oder weniger langem Stehen kristallin erstarren. Ihre Schmelzpunkte liegen um und über 100 °C. Für die Verarbeitung von Rohstoffen bei Raumtemperatur, wie für die Schaumstofftechnik erforderlich, sind diese daher weniger geeignet.

Die Herstellung der erfindungsgemäß einzusetzenden Trishydroxyalkyl-mono-harnstoffe kann dadurch erfolgen, daß

a) Harnstoff mit einem Gemisch der Amine $HN(A{-}OH)_2$ und $H_2NA{-}OH$ oder aber stufenweise
b) aus

$$NH_2-\underset{\underset{O}{\|}}{C}-NHAOH \quad \text{und} \quad HN(A-OH)_2$$ (bevorzugtes Verfahren)

c) aus

$$NH_2-\underset{\underset{O}{\|}}{C}-N(A-OH)_2 \quad \text{und} \quad H_2NAOH$$ (bevorzugtes Verfahren)

umgesetzt wird, wobei A die bereits angeführte Bedeutung haben kann.

Beispiele für die als Flammschutzmittel für Polyurethankunststoffe wirkenden Hydroxyalkylharnstoffe sind :

$$(HO-CH_2-CH_2)_2N-\overset{\overset{O}{\|}}{C}-NH-CH_2-CH_2-OH$$

(Fortsetzung)

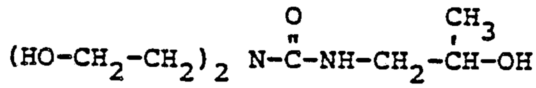

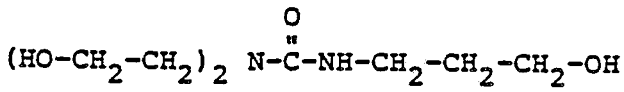

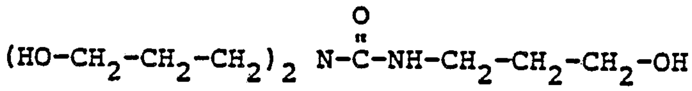

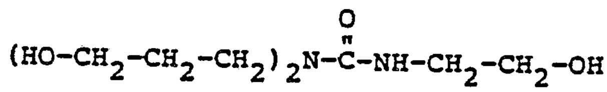

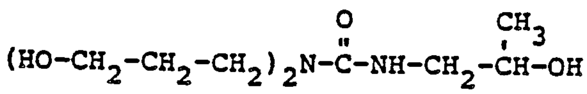

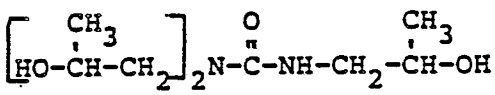

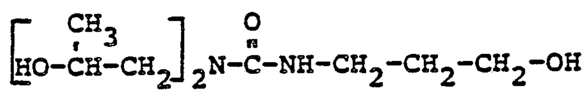

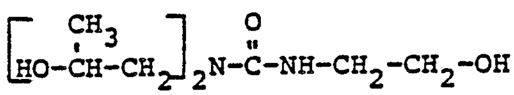

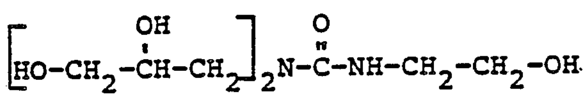

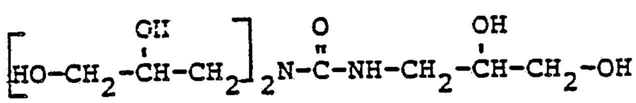

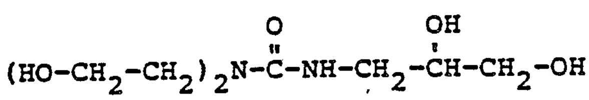

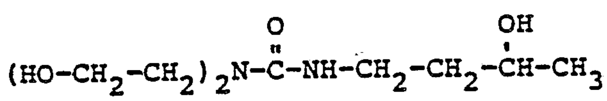

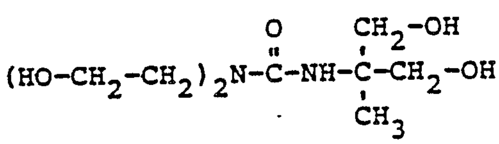

(Fortsetzung)

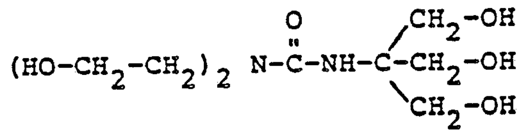

Für die Reihe der Hydroxyethyl-harnstoffe läßt sich folgende Reiehenfolge ihrer Flammschutzwirksamkeit aufstellen :

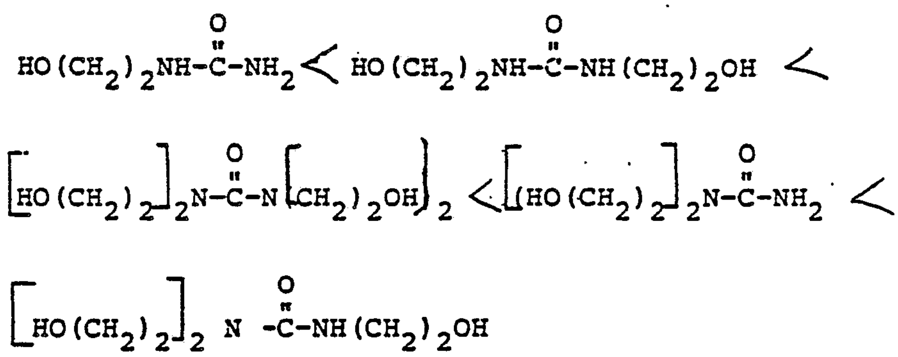

Überraschend ist hierbei, daß der Tris-hydroxyethylharnstoff das beste Flammschutzergebnis bringt und der Tetra-hydroxyethyl-harnstoff demgegenüber schlechter ist.

Dies gilt, wohlbemerkt, ohne die Berücksichtigung des Kristallisationsverhaltens der angeführten Produkte. Hierdurch sind, wie schon erwähnt, der Verarbeitbarkeit von symmetrischen als auch asymmetrischen Bis-hydroxyethyl-harnstoffen Grenzen gesetzt.

Die Produkte, deren flüssige Vertreter für eine problemlose Verarbeitung natürlich besonders bevorzugt sind, werden in Mengen von 3 bis 100, bevorzugt 11,5 bis 50, besonders bevorzugt 11,5 bis 30 Teilen, bezogen auf 100 Teile der höhermolekularen Verbindungen mit mindestens 2, gegenüber Isocyanaten reaktionsfähigen Wasserstoffatomen, vorzugsweise höhermolekularen Polyolen, vom Molekulargewicht 400 bis 10.000, zur Herstellung der PU-Schaumstoffe eingesetzt. Bevorzugte Umsetzungsformen sind 3-50 Gew.-%ige Suspensionen oder Dispersionen der Mono-harnstoffe in den höhermolekularen Verbindung. Es können auch beliebige Gemische von Tris-hydroxyalkyl-mono-harnstoffen eingesetzt werden. Besonders bevorzugt sind Dispersionen der Tris-hydroxyalkyl-mono-harnstoffe, insbesondere in mehr als 2-funktionellen Polyethern.

Zur Herstellung von Polyurethanschaumstoffen unter Mitverwendung der erfindungsgemäßen Flammschutzmittel vom Typ der Poly-hydroxyalkyl-harnstoffe werden als schaumstoffbildende Ausgangsmaterialien eingesetzt :

1. Verbindungen mit mindestens zwei gegenüber Isocyanaten reaktionsfähigen Wasserstoffatomen von einem Molekulargewicht in der Regel von 400 bis 10.000. Hierunter versteht man neben Aminogruppen, Thiolgruppen oder Carboxylgruppen aufweisenden Verbindungen vorzugsweise Hydroxylgruppen aufweisende Verbindungen, insbesondere 2 bis 8 Hydroxylgruppen aufweisende Verbindungen, speziell solche vom Molekulargewicht 600 bis 6.000, vorzugsweise 1.500 bis 4.000, vorzugsweise aber 2 bis 4 Hydroxylgruppen aufweisende Polyester, Polyether, Polythioether, Polyacetale, Polycarbonate und Polyesteramide, wie sie für die Herstellung von homogenen und zellförmigen Polyurethanen an sich bekannt sind und wie sie z. B. in der DE-A 2 832 253, Seiten 11 bis 18, beschrieben werden. Besonders bevorzugt sind Polyether, die durch Addition von einem oder mehreren Alkylenoxiden (Ethylenoxid und besonders Propylenoxid) an zwei- oder mehrwertige « Starter » (Propylenglykol, Glycerin, Sorbit, Formose, Triethanolamin, Trimethylolpropan) erhalten werden, sowie Polyether, welche Polyadditionsprodukte aus Diisocyanaten und Hydrazin und/oder Diaminen und/oder Glykolen oder Polymerisate und/oder Pfropfpolymerisate, vorzugsweise aus Styrol und Acrylnitril dispergiert oder gelöst enthalten. Die bevorzugten Polyether haben dabei eine mittlere Funktionalität über 2,0.

2. Gegebenenfalls werden als Ausgangsmaterialien Verbindungen mit mindestens 2 gegenüber Isocyanaten reaktionsfähigen Wasserstoffatomen und einem Molekulargewicht von 18 bis 399 mitverwendet. Auch in diesem Fall versteht man hierunter Hydroxylgruppen und/oder Aminogruppen und/oder Thiolgruppen und/oder Carboxylgruppen und/oder Hydrazidgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gegenüber Isocyanatten reaktionsfähige Wasserstoffatome auf. Beispiele werden hierfür in der

DE-A 2 832 253, Seiten 19-20, beschrieben. Genannt seien Wasser, Hydrazin, Ethylenglykol, Butandiol-1,4, Trimethylolpropan, Formit-Gemische oder Adipinsäuredihydrazid.

3. Aliphatische, cycloaliphatische, araliphatische, heterocyclische und besonders aromatische Polyisocyanate, wie sie z. B. von W. Siefken in Justus Liebigs Annalen der Chemie, 562, Seiten 75-136, beschrieben werden, beispielsweise solche der Formel $Q(NCO)_n$, in der n = 2 bis 4, vorzugsweise 2 und Q einen aliphatischen Kohlenwasserstoffrest mit 2 bis 18, vorzugsweise 6 bis 12 C-Atomen, einen cycloaliphatischen Kohlenwasserstoffrest mit 4 bis 20, vorzugsweise 5 bis 11 C-Atomen, einen aromatischen Kohlenwasserstoffrest mit 6 bis 20, vorzugsweise 6 bis 13 C-Atomen, oder einen araliphatischen Kohlenwasserstoffrest mit 8 bis 15, vorzugsweise 8 bis 13 C-Atomen bedeuten, z. B. solche Polyisocyanate wie sie in der DE-A 2 832 253, Seiten 10 bis 11 beschrieben werden. Besonders bevorzugt werden die technisch leicht zugänglichen Polyisocyanate, z. B. das 2,4- und/oder 2,6-Toluylendiisocyanat sowie beliebige Gemische dieser Isomeren (« TDI »), Diphenylmethan-diisocyanate (4,4'- und/oder 2,4'- und/oder 2,2'-Isomere), Polyphenyl-polymethylen-polyisocyanate, wie sie durch Anilin-Formaldehyd-Kondensation und anschließende Phosgenierung hergestellt werden (« rohes MDI ») und « modifizierte Polyisocyanate », die z. B. Carbodiimidgruppen, Urethangruppen, Allophanatgruppen, Isocyanuratgruppen, Harnstoffgruppen und/oder Biuretgruppen aufweisen ; insbesondere solche modifizierten Polyisocyanate, die sich vom 2,4- und/oder 2,6-Toluylendiisocyanat und bevorzugt vom 4,4'- und/oder 2,4'-Diphenylmethandiisocyanat ableiten. Werden nur zweifunktionelle höhermolekulare Verbindungen und gegebenenfalls nur zweifunktionelle weitere, niedermolekulare Kettenverlängerungsmittel verwendet, so setzt man bevorzugt modifizierte Polyisocyanate mit einer Funktionalität von mehr als 2,0 ein bzw. verwendet Tri- und/oder höherfunktionelle Poly-isocyanate. Gegebenenfalls werden Hilfs- und Zusatzmittel wie leichtflüchtige anorganische, jedoch bevorzugt organische Substanzen als Treibmittel, Katalysatoren der an sich bekannten Art, wie tertiäre Amine, Zinn-(II)- und Zinn-(IV)-Verbindungen, oberflächenaktive Zusatzstoffe, wie Emulgatoren und Schaumstabilisatoren, Reaktionsverzögerer, z. B. sauer reagierende Stoffe wie Salzsäure oder organische Säurehalogenide oder Säure, ferner Zellregler der an sich bekannten Art wie Paraffine, Fettalkohole oder Dimethylpolysiloxane sowie Pigmente oder Farbstoffe der an sich bekannten Art, ferner Stabilisatoren gegen Alterungs-, Licht- und Witterungseinflüsse, Weichmacher und fungistatisch und bakteriostatisch wirkende Substanzen, sowie Füllstoffe zugesetzt. Diese gegebenenfalls mitzuverwendenden Hilfs- und Zusatzstoffe werden beispielsweise in der DE-A 2 732 292, Seiten 21 bis 24 ausführlich beschrieben. Weitere Beispiele der Hilfs- und Zusatzmittel werden im Kunststoffhandbuch, Band VII, herausgegeben von Vieweg und Hoechteln, Carl-Hanser-Verlag, München 1966, auf den Seiten 103-113 beschrieben.

Die erfindungsgemäßen, als einbaufähige Flammschutzmittel wirkenden Tris-hydroxyalkyl-harnstoffe, werden, üblicherweise mit höhermolekularen, vorzugsweise Polyol-Verbindung in den angegebenen Mengen vermischt. Es entstehen feinteilige Suspensionen fester Verbindungen in den höhermolekularen Polyolen oder vorzugsweise in flüssiger Form dispergierte Polyhydroxylverbindungen ; gegebenenfalls werden die Poly-hydroxyalkyl-harnstoffe bei Temperaturen oberhalb ihrer Schmelzpunkte in den höhermolekularen Polyolen dispergiert. Das Gemisch kann weiterhin die üblichen Zusatz- und Hilfsstoffe für die Schaumstoffherstellung enthalten. Die komplette Rohstoff-Abmischung ist im Rahmen überlicher Garantiezeiten lagerstabil.

Eine bevorzugte Verwendungsform liegt in 3 bis 50 gew.-%igen (besonders 10-30 gew.-%igen) Suspensionen oder Dispersionen der Tris-hydroxyalkyl-mono-harnstoffe in den höhermolekularen Verbindungen mit mindestens 2, gegenüber NCO reaktiven Endgruppen aus der Reihe von Hydroxyl-, primären und/oder sekundären Amino-, Carboxyl- oder Hydrazidgruppen, mit Molekulargewichten von 400 bis 10.000, vorzugsweise in höhermolekularen Polyolen.

Hauptanwendungsgebiet für den Einbau der Flammschutzmittel ist die Herstellung von Integralschaumstoffen sowie von halbharten und harten Hartschaumstoffen. Bevorzugt werden dabei Polyurethanschaumstoff-Systeme verwendet, welche organische Treibmittel anstelle von Wasser enthalten. Es können jedoch auch Wasser als Treibmittel enthaltende Polyurethanschaumstoff-Systeme verwendet werden, welche gegebenenfalls zu elastischen Schaumstoffen führen. Überraschenderweise hat sich herausgestellt, daß die erfindungsgemäß zu verwendenden Flammschutzmittel in wasserhaltigen, elastische Weichschaumstoffe ergebenden Systemen keinerlei zellöffnende Wirkung haben, wie dies für Poly-hydroxyalkyl-amid-Derivate mehrwertiger Carbonsäuren in der EP-A 68 281 beschrieben wurde.

Hauptanwendungsgebiete der erfindungsgemäß erhältlichen Produkte sind flammfeste, besonders halbharte oder harte Polyurethanschaumstoffe, welche z. B. für die Ausrüstung von Verkehrsmitteln geeignet sind, ferner sind es Hartschaumstoffe für Isolationsanwendungen und flammfeste Integralschaumstoffe für Formkörper, wie sie z. B. in der Autoindustrie Verwendung finden.

Die besondere Eignung der mit den erfindungsgemäßen Flammschutzmitteln ausgerüsteten Polyurethanschaumstoffe für die Innenausrüstung von Verkehrsmitteln wird durch spezielle Prüfmethoden nachgewiesen, und zwar durch MVSS 302, Bundesbahntest und Lufthansatest (FAR 25853). Hierauf wird im anwendungstechnischen Teil, Beispielteil C, ausführlich eingegangen.

Die erfindungsgemäßen Flammschutzmittel zeigen in den Polyurethanschaumstoffen bereits bei alleiniger Verwendung gute Wirksamkeit, wie die Brandergebnisse zeigen ; sie können jedoch auch in Kombination mit üblichen Flammschutzmitteln eingesetzt werden.

## Beispiele

Experimenteller Teil

A) Herstellung der Hydroxyalkyl-mono-harnstoffe

1. $NH_2-\overset{O}{\overset{\|}{C}}-N(CH_2-CH_2-OH)_2$ $C_5H_{12}N_2O_3$ (148)
CA: 23270-55-5

Aus 600 g (10 mol) Harnstoff und 1 050 g (10 mol) Diethanolamin durch Austreiben von Ammoniak bei 100-140 °C und Nachbehandlung im Wasserstrahlvakuum bei 80 °C, Ausbeute fast quantitativ ; das Reaktionsprodukt erstarrt innerhalb unterschiedlicher Zeiträume völlig kristallin ; OH-Zahl : 766, ber. 755 ;

2. $NH_2-\overset{O}{\overset{\|}{C}}-NH-CH_2-CH_2-OH$ $C_3H_8N_2O_2$ (104)
CA: 2078-71-9

Aus 600 g (10 mol) Harnstoff und 610 g (10 mol) Ethanolamin durch Austreiben von Ammoniak bei 80-120 °C und Nachbehandlung im Wasserstrahlvakuum bei 80 °C, Ausbeute fast quantitativ, OH-Zahl : 550, ber. 538 ;

3. $HO-CH_2-CH_2-NH-\overset{O}{\overset{\|}{C}}-NH-CH_2-CH_2-OH$ $C_5H_{12}N_2O$ (148)
CA: 15438-70-7

Aus 600 g (10 mol) Harnstoff und 1 220 g (20 mol) Ethanolamin durch Austreiben der entsprechenden Menge Ammoniak bei 80-120 °C, und Nachbehandlung im Wasserstrahlvakuum bei 80 °C, Ausbeute fast quantitativ, erstarrt nach kurzem Stehen ; OH-Zahl 786, ber. 755 ;

4. $(HO-CH_2-CH_2)_2N-\overset{O}{\overset{\|}{C}}-N(CH_2-CH_2-OH)_2$ $C_9H_{20}N_2O_5$ (236)
CA: 57462-27-8

Aus 600 g (10 mol) Harnstoff und 2 100 g (20 mol) Diethanolamin durch Austreiben von Ammoniak bei 100-140 °C und Nachbehandlung im Wasserstrahlvakuum bei 80 °C, Ausbeute 2 340 g, OH-Zahl 1 090, ber. 1 050, das Produkt bleibt flüssig, Viskosität : 4 000 mPas (25 °C) ;

5. $(HO-CH_2-CH_2)_2N-\overset{O}{\overset{\|}{C}}-NH-CH_2-CH_2-OH$ $C_7H_{16}N_2O_4$ (192)

(erfindungsgemäße Verbindung).

Aus 3 340 g (22,6 mol) N,N-Bis-2-hydroxyethylharnstoff nach Beispiel 1 und 1 380 g (22,6 mol) Ethanolamin durch Erhitzen auf 120 bis 140 °C und Nachbehandlung im Wasserstrahlvakuum bei 80 °C ; Ausbeute 4 200 g ; Viskosität 4 200 mPas bei 25 °C ; OH-Zahl 940, ber. 880 ;

6. $\left[HO-\overset{CH_3}{\overset{|}{C}H}-CH_2\right]_2N-\overset{O}{\overset{\|}{C}}-NH_2$ $C_7H_{16}N_2O_3$ (176)

Aus 150 g (2,5 mol) Harnstoff und 332,5 g (2,5 mol) Diisopropanolamin durch Erhitzen auf 115 bis 120 °C und Nachbehandlung im Wasserstrahlvakuum bei 80 °C, Ausbeute fast quantitativ, OH-Zahl 640, ber. 636. Das Produkt erstarrt nach kurzem Stehen wachsartig.

7. $(HO-CH_2-CH_2)_2N-\overset{O}{\overset{\|}{C}}-NH-CH_2-\overset{OH}{\overset{|}{C}H}-CH_3$ $C_8H_{18}N_2O_4$ (206)

(erfindungsgemäße Verbindung).

Aus 148 g (1 mol) N,N-Bis-2-hydroxyethyl-harnstoff nach Beispiel 1 und 75 g (1 mol) Aminopropanol-(2) durch Erwärmen auf 100-140 °C und Nachbehandlung im Wasserstrahlvakuum bei 80 °C ; Ausbeute quantitativ. Das Produkt bleibt flüssig ; Viskosität : 3 300 mPas (25 °C).

Analyse
ber. : C 46,5, H 8,7, N 13,6 ;
gef. : C 46,2, H 8,7, N 14,7 ;

8. 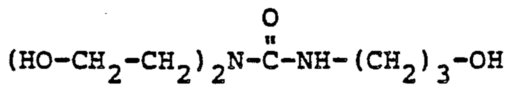 $C_8H_{18}N_2O_4$ (206)

(erfindungsgemäße Verbindung).

Aus 148 g (1 mol) N,N-Bis-2-hydroxyethyl-harnstoff nach Beispiel 1 und 75 g (1 mol) 3-Amino-1-propanol durch Erwärmen auf 100-140 °C und Nachbehandlung im Wasserstrahlvakuum bei 80 °C, Ausbeute quantitativ. Der Harnstoff bleibt flüssig ; Viskosität : 4 600 mPas (25 °C) ;

9. 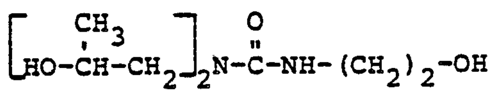 $C_9H_{20}N_2O_4$ (210)

Aus 176 g (1 mol) N,N-Bis-2-hydroxypropyl-harnstoff nach Beispiel 6 und 61 g Ethanolamin durch Austreiben von Ammoniak bei 120-140 °C und Nachbehandlung im Wasserstrahlvakuum bei 80 °C, Ausbeute quantitativ. OH-Zahl 770, ber. 763

10. 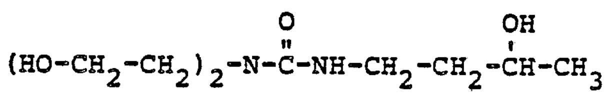 $C_8H_{20}N_2O_4$ (220)

(erfindungsgemäße Verbindung).

Aus 148 g (1 mol) N,N-Bis-2-hydroxyethyl-harnstoff nach Beispiel 1 und 89 g (1 mol) 4-Amino-2-butanol bei 120 bis 140 °C und Nachbehandlung im Wasserstrahlvakuum bei 80 °C, Ausbeute quantitativ. OH-Zahl 780, ber. 763

11. 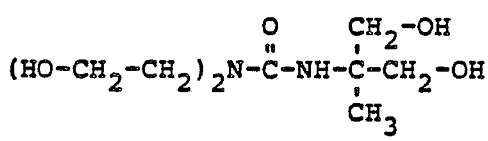 $C_9H_{20}N_2O_5$ (236)

Aus 148 g (1 mol) N,N-Bis-2-hydroxyethyl-harnstoff nach Beispiel 1 und 105 g (1 mol) 2-Amino-2-methyl-1,3-propandiol bei 120 bis 140 °C und Nachbehandlung im Wasserstrahlvakuum bei 80 °C, Ausbeute quantitativ. OH-Zahl 900, ber. 950

Viskosität : 57 000 mPas (25 °C)
ber . C 45,7, H 8,4, N 11,9
gef . C 45,4, H 8,3, N 12,3

12. 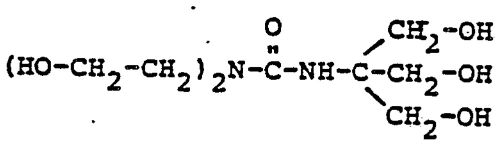 $C_9H_{20}N_2O_6$ (252)

Aus 74 g N,N-Bis-(2-hydroxyethyl)-harnstoff und 61 g 2-Amino-2-hydroxymethylpropandiol bei 120-140 °C und Nachbehandlung im Wasserstrahlvakuum bei 80 °C ; Ausbeute quantitativ, OH-Zahl 1 110, ber. , OH-Zahl gef. 1 150.

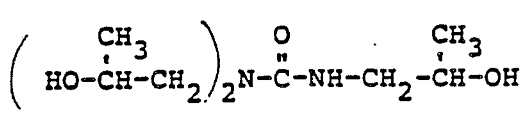 $C_{10}H_{22}N_2O_3$ (210)

(erfindungsgemäße Verbindung).

Aus 528 g (3,0 m) N,N-Bis-2-hydroxypropyl-harnstoff nach Beispiel 6 und 225 g (3 m) Aminopropanol-(2), durch Erwärmen auf 120-140 °C und Nachbehandlung im Wasserstrahlvakuum bei 80 °C, Ausbeute 694 g,

ber . C 51,2, H 9,4, N 11,95
gef . C 50,7, H 8,5, N 12,4
Viskosität : 4 500 mPas (25 °C).

Die Verbindungen 1 bis 4 und 6 stellen Vergleichssubstanzen dar, die in den nachstehenden Verwendungsbeispielen ebenfalls eingesetzt worden sind.

B) Verwendung zur Herstellung von Integral- bzw. Hartschaumstoffen

In allen Beispielen wurde die folgende Grundrezeptur verwendet ; die jeweiligen Flammschutzkomponenten sind in der Tabelle 2 aufgeführt :

Die Polyolgrundkomponente des Gemischs bestand aus :

100 Gew.-Teilen eines trifunktionellen Polyetherpolyols der OH-Zahl 35, welches durch Alkoxylierung von Trimethylolpropan unter Verwendung von Propylenoxid und anschließendes Aufpfropfen von ca. 15 %, bezogen auf die Gesamtmenge an Ethylenoxid hergestellt worden war,

9 Gew.-Teilen Ethylenglykol,

0,4 Gew.-Teilen Diazabicyclooctan (33 %ig in Diisopropanol, « 33 LV », Produkt der Houdry/Hüls in Marl, D 4370),

14 Gew.-Teilen Trichlorofluormethan.

Die Polyisocyanatkomponente bestand aus der in der Tabelle angegebenen Menge eines mit Dipropylenglykol modifizierten, flüssigen, im wesentlichen bifunktionellen Polyisocyanatgemischs der Diphenylmethanreihe mit einem NCO-Gehalt von 28 % und einer Viskosität bei 25 °C von 130 mPa.s.

Es wurden jeweils 100 Teile der Dispersion oder Suspension der Hydroxyalkyl-mono-harnstoffe in der Polyol-Grundkomponente mit der jeweils in Tabelle 1 angegebenen Isocyanatmenge verschäumt.

Die Polyol-Grundkomponente wird mit den jeweiligen, erfindungsgemäßen Flammschutzkomponenten vom Typ der Hydroxyalkyl-mono-harnstoffe in den in der Tabelle angegebenen Mengen bei Raumtemperatur oder gegebenenfalls erhöhter Temperatur verrührt. Es werden dabei leicht trübe Dispersionen der Hydroxyalkyl-harnstoffe in den Polyolen erhalten, welche eine hinreichende Stabilität (Standzeiten bis zu mehreren Monaten) aufweisen. Feste Hydroxyalkyl-mono-harnstoffe können auch mit hochwirksamen, zerkleinernd wirkenden Mischgeräten in Suspensionen in den höhermolekularen Polyolen überführt werden.

(Siehe Tabellen Seite 10 ff.)

Tabelle 1

| Flammschutzmittel (FSM) | Menge an FSM (in Gew.-t.) | Polyol-Grundkomponente (Gew.-t.) | FSM in Gew.-t. pro 100 Gew.-tle höhermolekularen Polyol | Isocyanatmenge (Gew.-t.) | Nachaktivierung mit Katalysator 33 LV |
|---|---|---|---|---|---|
| Tris-(chlorethyl)-phosphat (Vergleich) | 10 | 100 | | 43 | - |
| Verb.gem.Vorschrift 2* | 10 | 90 | 11.1 | 52 | - |
| " " " 3* | 15 | 85 | 17.6 | 61 | 1.0 |
| " " " 6* | 10 | 90 | 11.1 | 54 | - |
| " " " 1* | 20 | 80 | 25.0 | 66 | - |
| " " " 4* | 15 | 85 | 17.6 | 70 | 0.4 |
| " " " 5 | 10 | 90 | 11.1 | 60 | - |
| " " " 8 | 20 | 80 | 25.0 | 68 | - |
| " " " 7 | 15 | 85 | 17.6 | 63 | - |
| " " " 13 | 20 | 80 | 25.0 | 65 | - |
| " " " 13 | 15 | 85 | 17.6 | 60 | - |

(*) Vergleichsversuche.

Tabelle 2

Brandverhalten durch Einbau von Hydroxyalkyl-harnstoffen als Flammschutzmittel in Schaumstoffe mit einer Zusammensetzung entsprechend Tabelle 1.

| Flammschutzmittel (FSM) | MVSS 302 Klassifizierung | FAR 258536 vertikal zerstörte Probenlänge (mm) | Nachbrennzeit (sec) | Tropfenbrennzeit (sec) | bestanden | Bundesbahntest DV 899/35 Brennbarkeitsgrad | Tropfbarkeitsgrad |
|---|---|---|---|---|---|---|---|
| Tris-(chlorethyl)-phosphat (Vergleich) | SE | 45 | 32 | 15 | nein | B 3 | T 1 |
| FSM gem. Vorschrift 2 * | SE | 50 | 11 | 23 | nein | B 1 | T 1 |
| " " " 3 * | SE | 50 | 11 | 20 | nein | B 2 | T 1 |
| " " " 6 * | SE | 65 | 18 | 20 | nein | B 2 | T 1 |
| " " " 1 * | SE | 42 | 0 | 0 | ja | B 3 | T 1 |
| " " " 4 * | SE | 33 | 4 | 2 | ja | B 2 | T 1 |
| " " " 5 | SE | 35 | 0 | 0 | ja | B 3 | T 1 |
| " " " 8 | SE | 50 | 3 | 1 | ja | B 3 | T 1 |
| " " " 7 | SE | 40 | 0 | 0 | ja | B 3 | T 1 |
| " " " 13 | SE | 33 | 0 | 0 | ja | B 3 | T 1 |
| " " " 13 | SE | 37 | 0 | 0 | ja | B 3 | T 1 |

(*) Vergleichsversuche.

Die Beispiele zeigen, daß die erfindungsgemäßen Hydroxyalkyl-harnstoffe in den Brennbarkeitstests gemäß FAR 25853b mit der genauen Erfassung von zerstörter Probenlänge, Nachbrennzeit und Tropfenbrennbarkeit den Ergebnissen üblicher Flammschutzmittel mit den schon geschilderten Nachteilen noch überlegen sind.

Dies geht besonders aus den Beispielen 5, 8, 7, 13 der Trishydroxyalkylharnstoffe hervor.

C) Prüfung des Brandverhaltens

Fast alle Brennbarkeitstests sind an Risikoaspekten bestimmter Anwendungen orientiert. Für die verschiedenen Anwendungsgebiete existieren daher unterschiedliche Prüfverfahren, die zu Ergebnissen führen, welche miteinander nur bedingt vergleichbar sind.

Entsprechend der Verwendung der Polyurethanschaumstoffe wurden für die Untersuchung der Wirksamkeit der Flammschutzmittel drei Brandtests ausgewählt.

a) Prüfvorschrift dür Kfz-Innenausstattung gemäß DIN 75 200, entspricht den Federal Motor Vehicle Safety Standards (MVSS) 302 der USA.

b) Brennbarkeits-Test der Bundesbahn.

c) Brandverhalten von Luftfahrt-Werkstoffen : Federal Aviation Regulations (FAR) 25853 (USA).

Die Ausmaße und Anzahl der Prüfkörper, die Probenanordnnung, die Durchführung der Prüfung und die dabei zu erfüllende Forderung ist in der folgenden Aufstellung stichwortartig zusammengefaßt :

a) MVSS 302 (Docket 3-3) (DIN 75 200)
Prüfkörper : 3 Platten 350 × 100 × 13 mm
Meßmarken bei 38, 254 und 312 mm
Probenanordnung : horizontal
Beflammungsdauer : 15 sec
Bestimmung der Brandgeschwindigkeit v zwischen der 2. und 3. Markierung.
Forderung : v ≤ 100 mm/min
Klassifizierung : SE (selbstverlöschend), SE/NBR (keine Brenngeschwindigkeit, Probe verlöscht innerhalb 60 sec), BR (brennend, Geschw. wird angegeben).

b) Bundesbahn-Test DV 899/35
Prüfkörper : 3 Platten 300 × 100 × 30 mm
Probenanordnung : vertikal
Beflammungsdauer : 3 min
Bestimmung des Brennbarkeitsgrades (% verbrannte Fläche)
B 4 : nicht brennbar
B 3 : schwer entflammbar, bis 75 % verbrannte Fläche
B 2 : brennbar, 76-90 % verbrannte Fläche
B 1 : leicht brennbar, 91-100 % verbrannte Fläche
Bestimmung des Tropfbarkeitsgrades
T 4 : verformt und erweicht nicht, bildet keine Tropfen
T 3 : verformt stark, erweicht oder bildet Fäden
T 2 : tropft nichtbrennend ab
T 1 : tropft brennend ab und brennt weiter
Bestimmung des Qualmentwicklungsgrades
Q 4 : qualmt nur sehr wenig, 10 % Sichttrübung
Q 3 : qualmt und rußt mäßig, 11-40 % Sichttrübung
Q 2 : qualmt und rußt stark, 41-70 % Sichttrübung
Q 1 : qualmt und rußt sehr stark, 71-100 % Sichttrübung

c) Lufthansa-Test FAR 25 853
Probenanordnung : horizontal und vertikal
Prüfkörper horizontal : 3 Platten 350 × 100 × 13 mm
Prufkörper vertikal : 3 Platten 350 × 75 × 13 mm
Beflammungsdauer horizontal : 15 sec
Beflammungsdauer vertikal : 12 sec
Geforderte Meßwerte
horizontal : Brenngeschwindigkeit ≤ 63,4 mm/min
vertikal : Verbrennungslänge ≤ 203 mm
Nachbrennzeit ≤ 13 sec
Tropfenbrennzeit ≤ 5 sec

**Patentansprüche**

1. Verwendung von Hydroxyalkylharnstoffen als einbaufähige Flammschutzmittel bei der Herstellung von flammgeschützten Polyurethanschaumstoffen, bei der Verbindungen mit gegenüber Isocyanatgruppen reaktionsfähigen Wasserstoffatomen des Molekulargewichtsbereichs 400-10 000 verwendet werden, wobei die Hydroxyalkylharnstoffe in einer Menge von 3 bis 100 Gewichtsteilen, bezogen auf 100 Gewichtsteile diese Verbindungen mit gegenüber Isocyanatgruppen reaktionsfähigen Gruppen eingesetzt werden, dadurch gekennzeichnet, daß man als Hydroxyalkylharnstoffe Tris-hydroxyalkyl-mono-harnstoffe der allgemeinen Formel

$$HO-A-NH-\overset{O}{\overset{\|}{C}}-N(-A-OH)_2$$

verwendet, in welcher A für geradkettige oder verzweigte, gegebenenfalls eine oder mehrere OH-Gruppen aufweisende $C_2$-$C_6$-Alkylengruppen darstellt.

2. Verwendung gemäß Anspruch 1, dadurch gekennzeichnet, daß man die Hydroxyalkyl-harnstoffe in Form von 3 bis 50 gewichts-%-igen Dispersionen oder Suspensionen in den höhermolekularen Verbindungen mit gegenüber Isocyanatgruppen reaktionsfähigen Wasserstoffatomen einsetzt.

3. Verwendung gemäß Anspruch 1 und 2, dadurch gekennzeichnet, daß man als Hydroxyalkylharnstoffe solche der in Anspruch 1 genannten allgemeinen Formel verwendet, für welche A für Ethylen- und/oder 1,2-Propylengruppen steht.

4. Dispersionen oder Suspensionen von Tris-hydroxyalkyl-mono-harnstoffen der allgemeinen Formel

$$HO-A-NH-\overset{O}{\overset{\|}{C}}-N(-A-OH)_2$$

in welcher A die in Anspruch 1 genannte Bedeutung hat in höhermolekularen Verbindungen mit mindestens zwei gegenüber Isocyanatgruppen reaktionsfähigen Endgruppen aus der Reihe der Hydroxyl-, primären oder sekundären Amino-, Carboxyl- oder Hydrazid-Endgruppen mit Molekulargewichten von 400 bis 10 000, wobei der Gehalt der Hydroxyalkylharn stoffe 3 bis 50 Gewichtsprozent ausmacht.

5. Tris-hydroxyalkyl-mono-harnstoffe der allgemeinen Formel

$$HO-A-NH-\overset{O}{\overset{\|}{C}}-N(-A-OH)_2$$

in welcher A die Anspruch 1 genannte Bedeutung hat, mit Ausnahme von N,N-Bis(2-hydroxypropyl)-N'-(2-hydroxyethyl)-mono-harnstoff.

6. Tris-hydroxyalkyl-mono-harnstoffe nach Anspruch 5, dadurch gekennzeichnet, daß in der allgemeinen Formel A für eine Ethylen- und/oder eine 1,2-Propylengruppe steht.

**Claims**

1. Use of hydroxyalkylureas as incorporable flame retardants for the production of flame-resistant polyurethane foams, in which compounds in the molecular weight range of from 400 to 10 000 containing isocyanate reactive hydrogen atoms are used, the hydroxyalkylureas being used in a quantity of from 3 to 100 parts by weight, based on 100 parts by weight of these compounds which contain isocyanate reactive groups, characterised in that the hydroxyalkylureas used are tris-hydroxyalkylmonoureas corresponding to the following general formula

$$HO-A-NH-\overset{O}{\overset{\|}{C}}-N(-A-OH)_2$$

wherein A denotes straight chained or branched $C_2$-$C_6$-alkylene groups optionally containing one or more OH groups.

2. Use according to Claim 1, characterised in that the hydroxyalkylureas are used in the form of 3 to 50 % by weight dispersions or suspensions in the relatively high molecular weight compounds which contain isocyanate reactive hydrogen atoms.

3. Use according to claims 1 and 2, characterised in that the hydroxyalkylureas used are compounds corresponding to the general formula indicated in Claim 1 in which A stands for ethylene and/or 1,2-propylene groups.

4. Dispersions or suspensions of tris-hydroxyalkylmonoureas corresponding to the following general formula

$$HO-A-NH-\overset{\overset{\displaystyle O}{\|}}{C}-N(-A-OH)_2$$

wherein A has the meaning indicated in claim 1 in relatively high molecular weight compounds in the molecular weight range of from 400 to 10 000 containing at least two isocyanate reactive end groups selected from hydroxyl, primary or secondary amino, carboxyl and hydrazide end groups, the hydroxyalkylurea content amounting to 3 to 50 % by weight.

5. Tris-hydroxyalkyl-monoureas corresponding to the following general formula

$$HO-A-NH-\overset{\overset{\displaystyle O}{\|}}{C}-N(-A-OH)_2$$

wherein A has the meaning indicated above with the exclusion of N,N-bis-(2-hydroxypropyl)-N'-(2-hydroxyethyl)-monourea.

6. Tris-hydroxyalkyl-monoureas according to claim 5, characterised in that the symbol A in the general formula stands for an ethylene and/or a 1,2-propylene group.

**Revendications**

1. Utilisation d'hydroxyalcoyl-urées comme agents ignifugeants incorporables dans la fabrication de matières cellulaires en polyuréthanes ignifugées, dans laquelle on utilise des composés ayant des atomes d'hydrogène capables de réagir avec les isocyanates, et qui ont un intervalle de poids moléculaires de 400 à 10 000, les hydroxyalcoyl-urées étant utilisées en une quantité de 3 à 100 parties en poids par rapport à 100 parties en poids desdits composés avec groupes capables de réagir avec les groupes isocyanate, caractérisée en ce qu'on utilise comme hydroxyalcoyl-urées des tris-hydroxyalcoyl-mono-urées de formule générale :

$$HO-A-NH-\overset{\overset{\displaystyle O}{\|}}{C}-N(-A-OH)_2$$

dans laquelle A représente des groupes alcoylène en $C_2$ à $C_6$ à chaîne droite ou ramifiée présentant éventuellement un ou plusieurs groupes OH.

2. Utilisation selon la revendication 1, caractérisée en ce qu'on utilise les hydroxyalcoyl-urées sous la forme de dispersions ou de suspensions à 3 à 50 % en poids dans des composés à poids moléculaire élevé ayant des atomes d'hydrogène capables de réagir avec les groupes isocyanate.

3. Utilisation selon les revendications 1 et 2, caractérisée en ce qu'on utilise comme hydroxyalcoyl-urées celles de formule générale citée à la revendication 1 dans laquelle A représente des groupes éthylène et/ou 1,2-propylène.

4. Dispersions ou suspensions de tris-hydroxyalcoyl-mono-urées de formule générale :

$$HO-A-NH-\overset{\overset{\displaystyle O}{\|}}{C}-N(-A-OH)_2$$

dans laquelle A a la signification indiquée à la revendication 1, dans des composés à poids moléculaire élevé qui ont au moins deux groupes terminaux capables de réagir avec les groupes isocyanate appartenant à la série des groupes terminaux hydroxyle, amino primaires ou secondaires, carboxyle ou hydrazide, et qui ont des poids moléculaires de 400 à 10 000, la teneur des hydroxyalcoyl-urées représentant 3 à 50 % en poids.

5. Tris-hydroxyalcoyl-mono-urées de formule générale :

$$HO-A-NH-\overset{\overset{\displaystyle O}{\|}}{C}-N(-A-OH)_2$$

dans laquelle A a la signification citée à la revendication 1, à l'exception de la N,N-bis(2-hydroxypropyl)-N'-(2-hydroxyéthyl)-mono-urée.

6. Tris-hydroxyalcoyl-mono-urées selon la revendication 5, caractérisées en ce que dans la formule générale A représente un groupe éthylène et/ou 1,2-propylène.